# EUROPEAN PATENT SPECIFICATION

(11) **EP 2 680 857 B1**
(45) Date of publication and mention of the grant of the patent: **24.04.2019**
(21) Application number: 12707503.4
(22) Date of filing: 24.02.2012
(51) Int. Cl.: A61K 31/685, A61P 31/10

(54) **OLEYL PHOSPHOCHOLINE FOR THE TREATMENT OF MYCOSIS**
OLEYL-PHOSPHOCHOLIN ZUR BEHANDLUNG VON MYKOSE
OLÉYL-PHOSPHOCHOLINE POUR LE TRAITEMENT DES MYCOSES

(30) Priority: 04.03.2011 WO PCT/EP2011/053345
(43) Date of publication of application: 08.01.2014
(73) Proprietor: Oblita Therapeutics BVBA, 2980 Zoersel (BE)
(72) Inventor: JANSEN, Frans Herwig, B-2360 Oud-Turnhout (BE); FORTIN, Annie Marie, Québec, Québec H1T 3H3 (CA)
(74) Representative: Nederlandsch Octrooibureau
(86) International application number: PCT/EP2012/053144
(87) International publication number: WO 2012/119870

(56) References cited:
- EP-A2- 0 108 565
- WO-A2-2007/071658
- US-A1- 2010 144 676
- Wickham T Mok ET AL: J. Clin. Microbiol, 1 January 1997 (1997-01-01), page 2654, XP055125745, Retrieved from the Internet: URL:http://jcm.asm.org/content/35/10/2654. full.pdf [retrieved on 2014-06-27]
- Adela Enache-Angoulvant ET AL: "Invasive Saccharomyces Infection @BULLET CID 2005:41 (1 December) @BULLET 1559 Invasive Saccharomyces Infection: A Comprehensive Review", , 1 January 2005 (2005-01-01), XP055125749, Retrieved from the Internet: URL:http://cid.oxfordjournals.org/content/ 41/11/1559.full.pdf#page=1&view=FitH [retrieved on 2014-06-27]
- A. K. Person ET AL: "Aspergillus niger: an unusual cause of invasive pulmonary aspergillosis", Journal of Medical Microbiology, vol. 59, no. 7, 18 March 2010 (2010-03-18) , pages 834-838, XP55125729, ISSN: 0022-2615, DOI: 10.1099/jmm.0.018309-0
- T. J. Walsh ET AL: "Molecular Detection and Species-Specific Identification of Medically Important Aspergillus Species by Real-Time PCR in Experimental Invasive Pulmonary Aspergillosis", JOURNAL OF CLINICAL MICROBIOLOGY, vol. 49, no. 12, 1 December 2011 (2011-12-01), pages 4150-4157, XP055516730, US ISSN: 0095-1137, DOI: 10.1128/JCM.00570-11

## Description

The present invention relates to the treatment of mycosis, and especially the treatment of virulent or invasive mycosis such as candidemia, cryptococcosis or aspergillosis.

Mycosis is a fungal infection of vertebrates, including humans. Mycosis is a common infection, and a variety of environmental and physiological conditions can contribute to the development of mycosis. Inhalation of fungal spores or localized colonization of the skin may initiate persistent infections and, accordingly, mycosis often starts in the lungs or on the skin.

Mycosis can be classified according to the tissue initially colonized.

Superficial mycosis is limited to the outermost layers of the skin and hair. An example of superficial mycosis is *Tinea versicolor,* a fungus infection that commonly affects young people.

Cutaneous mycosis extends deeper into the epidermis and also includes invasive hair and nail diseases. These diseases are restricted to the keratinized layers of the skin, hair, and nails. Unlike the superficial mycosis, host immune responses may be evoked resulting in pathologic changes expressed in the deeper layers of the skin.

Subcutaneous mycosis involves the dermis, subcutaneous tissues, muscle, and fascia. These infections are chronic and can be initiated by piercing trauma functioning as a port of entry for the fungi. These infections are difficult to treat and may require surgical interventions such as debridement.

Systemic mycosis generally starts with infection of the lungs, but also other ports of entry are known, and may spread to many organ systems and tissues. Pathogenic fungi causing systemic mycosis are inherently virulent.

A special class of systemic mycosis is systemic mycosis caused by opportunistic pathogens. Systemic mycosis caused by opportunistic pathogens is often an infection in patients with immune deficiencies. Examples of immunocompromised conditions include AIDS, alteration of normal flora by antibiotics, immunosuppressive therapy and metastatic cancer. Examples of opportunistic mycosis include candidiasis, cryptococcosis and aspergillosis.

Pathogenic fungi and especially pathogenic fungi causing systemic mycosis are, for example, fungi belonging to the genus *Candida,* the genus *Aspergillus* and the genus *Cryptococcus.*

*Candida* species, such as *Candida albicans,* are major human pathogens that are known for causing opportunist infections in immunocompromised hosts such as transplant patients, patients suffering from AIDS and cancer patients. Infections are difficult to treat and can be very serious, i.e. 30% to 40% of the systemic infections result in death.

Aerosolized *Aspergillus* spores are found nearly everywhere and constant exposure to *Aspergillus* is common. *Aspergillus* can generally cause serious mycosis in three ways: through the production of mycotoxins; through induction of allergenic responses; and through systemic infections.

The most common pathogenic species are *Aspergillus fumigatus* and *Aspergillus flavus. Aspergillus flavus* produces an aflatoxin which is both a toxin and a carcinogen.

Mycosis caused by *Aspergillus* is generally designated as aspergillosis. The symptoms of aspergillosis include fever, cough, chest pain or breathlessness.

*Cryptococcus neoformans* can cause a severe form of meningitis and meningo-encephalitis for example in HIV positive patients and AIDS. *Cryptococcus neoformans* is a major human and animal pathogen. *Cryptococcus laurentii* and *Cryptococcus albidus* have been known to cause moderate-to-severe disease in human patients with compromised immunity. *Cryptococcus gattii* can cause disease in non-immunocompromised people.

Presently, amphotericin B, voriconazole and itraconazole are amongst the most commonly used medicaments to combat severe forms of mycosis such as subcutaneous mycosis and systemic mycosis. However, serious side effects are associated with these drugs.

Amphotericin B is a polyene anti-fungal drug often used intravenously for systemic mycosis. It was originally extracted from *Streptomyces nodosus,* a filamentous bacterium, in 1955.

Two amphotericins, amphotericin A and amphotericin B are known, but only B is used clinically, because it is significantly more active *in vivo.* Amphotericin A is almost identical to amphotericin B (having a double C=C bond between the 27th and 28th carbon), but has little anti-fungal activity has been observed.

Currently, the drug is available as plain amphotericin B, a cholesteryl sulfate complex, a lipid complex, and as a liposomal formulation.

One of the main intravenous uses of amphotericin B is in treating various systemic mycoses, including cryptococcal meningitis, for example in critically ill, comorbidly infected or immunocompromised patients.

Another intravenous use of amphotericin B is as a drug of last resort in otherwise untreatable parasitic protozoan infections such as visceral leishmaniasis and primary amoebic meningoencephalitis.

Amphotericin B is well-known for its severe and potentially lethal side effects. Very often, a serious acute reaction after the infusion (1 to 3 hours later) is noted, consisting of high fever, shaking chills, hypotension, anorexia, nausea, vomiting, headache, dyspnea and tachypnea, drowsiness, and generalised weakness. To decrease the likelihood and severity of the symptoms, initial doses should be low, and increased slowly.

Intravenously administered amphotericin B has also been associated with multiple organ damage in therapeutic doses. Nephrotoxicity (kidney damage) is a frequently reported side effect and can be severe and/or irreversible.

Voriconazole (VFEND) is a triazole anti-fungal medication that is generally used to treat virulent or invasive mycosis. This type of mycosis is generally seen in patients who are immunocompromised, and includes invasive candidiasis, invasive aspergillosis, and certain emerging fungal infections.

Voriconazole has become the new standard of care in the treatment of invasive aspergillosis, which may occur in immunocompromised patients, including allogeneic BMT, other hematologic cancers, and solid organ transplants.

Voriconazole is better tolerated than amphotericin B, with fewer adverse effects and a longer duration of therapy.

Voriconazole has proven to be as effective as a regimen of intravenous amphotericin B followed by oral fluconazole in patients with culture-proven candidemia. Voriconazole cleared *Candida* yeast from the bloodstream as quickly as amphotericin B (median 2 days) and showed a trend toward better survival. Voriconazole is also associated with fewer serious adverse events and cases of renal toxicity, but a higher incidence of visual disturbances was observed.

Voriconazole has also been proven effective against a number of other serious fungal pathogens. These include infections by *Fusarium spp.* and *Scedosporium apiospermum* (asexual form of *Pseudallescheria boydii*)*.*

Although infrequently seen in the present clinical setting, these fungi are emerging as more common and deadly causes of fungal infection in immunocompromised patients, and the development of voriconazole has been an important advance in their treatment as they are generally resistant to other antifungal agents (including amphotericin B).

The most common side effects associated with voriconazole include transient visual disturbances, fever, rash, vomiting, nausea, diarrhea, headache, sepsis, peripheral edema, abdominal pain, and respiratory disorder. Also cases of serious hepatic reactions during treatment with voriconazole have been reported.

Itraconazole is a triazole anti-fungal agent that is prescribed to patients with mycosis. The drug may be given orally or intravenously.

Itraconazole has a broader spectrum of activity than fluconazole (but not as broad as voriconazole or posaconazole). In particular, it is active against *Aspergillus,* which fluconazole is not. It has been approved for the treatment of blastomycosis, histoplasmosis and onychomycosis. It is also prescribed for systemic infections such as aspergillosis, candidiasis and cryptococcosis where other antifungal drugs are inappropriate or ineffective.

Itraconazole is a relatively well-tolerated drug (although not as well tolerated as fluconazole or voriconazole) and the range of adverse effects it produces is similar to the other azole anti-fungals.

Elevated alanine aminotransferase levels are found in 4% of people taking itraconazole forming a risk of developing congestive heart failure. Other reported adverse effects of itraconazole are nausea, vomiting, abdominal pain, fatigue, loss of appetite, yellow skin (jaundice), yellow eyes, itching, dark urine, and/or pale stool.

Considering the above, there is a need in the art for anti-fungal agents being similarly effective as amphotericin B, voriconazole and/or itraconazole with respect to anti-fungal activity but which are better tolerated, i.e. with less, reduced or less severe side effects.

Further, considering the rapid development of resistance against known anti-fungal compounds, there is a continuing need in the art for further compounds with antifungal activity, and especially anti-fungal compounds effective against life threatening invasive or virulent fungal pathogens.

For example, patent application EP 0 108 565 relates to antifungal agents containing phospholipids. As an illustration, the antifungal properties of oleyl phosphocholine against *Trichophyton, Aspergillus niger, Penicillium* and yeasts have been described therein.

Accordingly, it is an object of the present invention, amongst other objects, to provide anti-fungal compounds meeting the above needs in the art.

According to the invention, this object, amongst other objects, according to a first aspect is met by appended claim 1.

Specifically, this object, amongst other objects, according to a first aspect is met by oleyl phosphocholine for use in the treatment of mycosis in a vertebrate, preferably for use in the treatment of invasive or virulent mycosis in a vertebrate.

Virulent mycosis as used herein indicates a primary fungal infection capable of extending, or spreading, the infected area of an organ or tissue infected.

Invasive mycosis as used herein indicates a primary fungal infection capable of further infecting, or spreading to, other organs or tissues.

In the present context, mycosis can be both virulent and invasive.

According to a preferred embodiment of this aspect of the present invention, the present mycosis is selected from the group consisting of superficial mycosis, cutaneous mycosis, subcutaneous mycosis, and systemic mycosis and especially invasive or virulent subcutaneous mycosis or invasive or virulent systemic mycosis.

According to another preferred embodiment of this aspect of the present invention, the present vertebrate is a human, preferably an immunocompromised patient.

According to one preferred embodiment of the present disclosure, the causative pathogen of the present mycosis is selected from the genus *Candida, Aspergillus, Fusarium, Cryptococcus, Microsporum, Sporothrix, Trichophyton* and *Scedosporium.*

According to an especially preferred embodiment of the present invention, the present oleyl phosphocholine is used for the treatment of mycosis caused pathogens selected from the group consisting of *Candida albicans, Candida parapsilosis, Candida glabrata, Candida krusei, Aspergillus fumigatus, Aspergillus terreus, Fusarium solani, Scedosporium prolifacans, Cryptococcus neoformans, Microsporum canis, Sporothrix schenkii, Trichophyton rubrum, Trichophyton mentagrophytes,* and *Fusarium oxysporum.*

According to another preferred embodiment of this aspect of the present invention, the present oleyl phosphocholine is formulated for parenteral, oral or topical administration. Especially in case the present mycosis is subcutaneous mycosis oleyl phosphocholine is formulated for topical or oral administration and in case the present mycosis is systemic mycosis oleyl phosphocholine is formulated intravenous, parenteral or oral administration.

According to an especially preferred embodiment of the present invention, the present oleyl phosphocholine is used for the treatment of a mycosis selected from the group consisting of candidemia, aspergillosis and cryptococcosis.

Considering the effective anti-fungal activity of oleyl phosphocholine, the present invention, according to another aspect, relates to methods for treatment of mycosis comprising administering to, or applying on a vertebrate suffering from mycosis, preferably invasive or virulent mycosis, a therapeutically effective dose of oleyl phosphocholine as further specified in the Claims.

According to a preferred embodiment of the present disclosure, the present mycosis is caused by a yeast or fungus belonging to a genus selected from the group consisting of *Candida, Aspergillus, Fusarium, Cryptococcus, Microsporum, Sporothrix, Trichophyton* and *Scedosporium.* According to a preferred embodiment of the invention, the present mycosis is caused by a pathogen selected from the group consisting of *Candida albicans, Candida parapsilosis, Candida glabrata, Candida krusei, Aspergillus fumigatus, Aspergillus terreus, Fusarium solani, Scedosporium prolifacans, Cryptococcus neoformans, Microsporum canis, Sporothrix schenkii, Trichophyton rubrum. Trichophyton mentagrophytes and Fusarium oxysporum.*

According to yet another preferred embodiment of this aspect, the present invention relates to the present use of oleyl phosphocholine formulated by parenteral, oral or topical administration.

According to a particularly preferred embodiment of this aspect, oleyl phosphocholine is used for the treatment of candidemia, aspergillosis and cryptococcosis.

The present invention will be further detailed and illustrated in the examples below representing particularly preferred embodiments of the present invention.

### Examples

### Example 1

The following ten strains were tested for assaying the anti-fungal activity of oleyl phosphocholine. One *Candida strain,* i.e. 19114, was selected because of its resistance against voriconazole and itraconazole

**Table 1: Strain tested for anti-fungal activity of oleyl phosphocholine**

| **Strain** | | |
|---|---|---|
| **Yeasts** | **strain isolate** | **remark** |
| *Candida albicans* | 3731 | ATCC 10231 |
| *Candida albicans* | 19114 | clinical blood isolate resistant for voriconazole and itraconazole |
| *Candida parapsilosis* | 3270 | ATCC 22019 |
| *Candida glabrata* | 19371 | clinical blood isolate |
| *Candida krusei* | 9560 | ATCC 6258 |

| **filamentous fungi** | | |
|---|---|---|
| *Aspergillus fumigatus* | 18963 | NCPF 7367 |
| *Aspergillus niger* | 5788 | ATCC 10864 |
| *Aspergillus terreus* | 13669 | clinical isolate |
| *Fusarium solani* | 18489 | clinical isolate |
| *Scedosporium prolifacans* | 22387 | clinical blood isolate |

The above strains were contacted with oleyl phosphocholine on microplates and the anti-fungal activity of oleyl phosphocholine was determined. In comparison, the same test was performed using amphotericin B (AMB), voriconazole (VRZ) and itraconazole (ITR).
The test protocol used was in conformity with the standard CLSI method:
Clinical and Laboratory Standards Institute/National Committee for Clinical Laboratory Standards (2002) Reference method for broth dilution antifungal susceptibility testing of yeasts. Approved Standard document M27-A2. Clinical and Laboratory Standards Institute/National Committee for Clinical Laboratory Standards Institute, Wayne PA.
Clinical and Laboratory Standards Institute/National Committee for Clinical Laboratory Standards. (2002) Reference method for broth dilution antifungal susceptibility testing of filamentous fungi. Approved Standard document M38-A. Clinical and Laboratory Standards Institute/National Committee for Clinical Laboratory Standards Institute, Wayne

The following concentrations of oleyl phosphocholine (Dafra), amphotericin B (AMB), voriconazole (VRZ) and itraconazole (ITR) were tested: 10, 5, 2.5, 1.25, 0.625, 0.312, 0.15, 0.078, 0.039, 0.019, 0.009, and 0.004 µg/ml

The results, after visual and spectrophometric reading after 48 hours of incubation, are presented as MIC50 and MIC 100, i.e. the minimum inhibitory concentration required inhibiting the growth of 50% or 100% of the microorganisms, respectively.

**Table 2: Minimum inhibitory concentration required inhibiting the growth of 50% or 100% of the microorganisms**

| **Yeast** | IHEM N° | Dafra-MIC100 (48 h) (µg/ml) | ITR (MIC50) (µg/ml) | VRZ (MIC50) (µg/ml) | AMB (MIC100) (µg/ml) |
|---|---|---|---|---|---|
| *Candida albicans* | 3731 | 2.5 | 0.032 | 0.064 | 0.5 |
| *Candida albicans* | 19114 | 2.5 | > 16 | > 16 | 0.5 |
| *Candida parapsilosis* | 3270 | 5 | 0.064 | 0.125 | 1 |
| *Candida glabrata* | 19371 | 5 | 0.5 | 0.25 | 0.5 |
| *Candida krusei* | 9560 | 5 | 0.25 | 0.5 | 1 |
| | | | | | |

| ***Fungi*** | IHEM N° | Dafra-MIC100 (48 h) (µg/ml) | ITR (MIC100) (µg/ml) | VRZ (MIC100) (µg/ml) | AMB (MIC 100) (µg/ml) |
|---|---|---|---|---|---|
| *Aspergillus fumigatus* | 18963 | 5 | 0.5 | 1 | 2 |
| *Aspergillus niger* | 5788 | 10 | 0.5 | 1 | 1 |
| *Aspergillus terreus* | 13669 | 10 | 0.25 | 0.5 | > 8 |
| *Fusarium solani* | 18489 | 5 | >16 | 16 | 2 |
| *Scedosporium prolificans* | 22387 | 5 | >16 | 16 | > 8 |

As can be seen in the above table, oleyl phosphocholine was capable of inhibiting the growth of yeast and fungi in a similar concentration range as the commonly used anti-fungal agents amphotericin B, voriconazole and itraconazole.

Further, the results for the resistant *Candida* strain 19114 show that oleyl phosphocholine provides a suitable alternative for the known anti-fungal agents against which resistance has been developed.

Of special interest is the anti-fungal activity of oleyl phosphocholine against *Fusarium solani* and *Scedosporium prolificans,* two emerging pathogens, against at least two of the known anti-fungal agents, i.e. voriconazole and itraconazole, are less effective.

### Example 2

The following strains were tested for assaying the anti-fungal activity of oleyl phosphocholine.

**Table 3: Strains used for assaying the anti-fungal activity of oleyl phosphocholine.**

| |
|---|
| *Candida albicans* |
| *Candida albicans B59630 (R)* |
| *Candida albicans B63195 (S)* |
| *Candida parapsilosis B66126* |
| *Cryptococcus neoformans* |
| *Cryptococcus neoformans B66663* |
| *Microsporum canis* |
| *Sporothrix schenkii* |
| *Sporothrix schenkii B62482* |
| *Trichophyton rubrum* |
| *Trichophyton rubrum B68183* |
| *Trichophyton mentagrophytes B70554* |
| *Aspergillus fumigatus* |
| *Aspergillus fumigatus B42928* |
| *Aspergillus fumigatus B19119* |
| *Fusarium solani IHEM22128* |
| *Fusarium oxysporum IHEM3014* |

The above strains were contacted oleyl phosphocholine on microplates and the anti-fungal activity of oleyl phosphocholine was determined.

Anti-fungal activity was expressed as IC₅₀, i.e. the concentration oleyl phosphocholine needed to kill 50% of the pathogens. The results are summarised in Table 4 below:

**Table 4: In vitro efficacy of OlPC against yeasts and fungi expressed as IC₅₀ concentration.**

| **Yeasts/Fungi** | **OlPC IC₅₀ (µM)** |
|---|---|
| *Candida albicans* | 7.89 |
| *Candida albicans B59630 (R)* | 8.00 |
| *Candida albicans B63195 (S)* | 7.77 |
| *Candida parapsilosis B66126* | 8.00 |
| *Cryptococcus neoformans* | 4.50 |
| *Cryptococcus neoformans B66663* | 0.50 |
| *Microsporum canis* | 1. 66 |
| *Sporothrix schenkii* | 8.16 |
| *Sporothrix schenkii B62482* | 1. 97 |
| *Trichophyton rubrum* | 5.87 |
| *Trichophyton rubrum B68183* | 2.00 |
| *Trichophyton mentagrophytes B70554* | 2.00 |
| *Aspergillus fumigatus* | 18.76 |
| *Aspergillus fumigatus B42928* | 8.00 |
| *Aspergillus fumigatus B19119* | 1. 87 |
| *Fusarium solani IHEM22128* | 2.31 |
| *Fusarium oxysporum IHEM3014* | 2.55 |

## Claims

1. Oleyl phosphocholine for use in the treatment of invasive or virulent mycosis in a vertebrate wherein the causative pathogen of said mycosis is selected from the group consisting of *Candida albicans, Candida parapsilosis, Candida glabrata, Candida krusei, Aspergillus fumigatus, Aspergillus terreus, Fusarium solani, Scedosporium prolifacans, Cryptococcus neoformans, Microsporum canis, Sporothrix schenkii, Trichophyton rubrum, Trichophyton mentagrophytes,* and *Fusarium oxysporum.*

2. Oleyl phosphocholine for use according to claim 1, wherein said vertebrate is a human, preferably an immunocompromised patient.

3. Oleyl phosphocholine for use according to claim 1 or claim 2, wherein said oleyl phosphocholine is formulated for parenteral, oral or topical administration.

4. Oleyl phosphocholine for use according to claim 1 or claim 2, wherein said mycosis is systemic mycosis and said formulation is for intravenous or oral administration.

5. Oleyl phosphocholine for use according to claim 1 or claim 2, wherein said mycosis is subcutaneous mycosis and said formulation is for topical or oral administration.

6. Oleyl phosphocholine for use according to any one of the claims 1 to 5, wherein said mycosis is selected from the group consisting of candidemia, aspergillosis and cryptococcosis.

## Patentansprüche

1. Oleylphosphocholin zur Verwendung bei der Behandlung von invasiver oder virulenter Mykose bei einem Wirbeltier, wobei das für die besagte Mykose ursächliche Pathogen aus der Gruppe ausgewählt ist, bestehend aus *Candia albicans, Candia parapsilosis, Candia glabrata, Candia krusei, Aspergillus fumigatus, Aspergillus terreus, Fusarim solani, Scedosporium prolifacans, Cryptococcus neoformans, Microsporum canis, Sporothrix schenkii, Trichopyton rubrum, Trichopyton mentagrophytes* und *Fusarium oxysporum.*

2. Oleylphosphocholin zur Verwendung nach Anspruch 1, wobei besagtes Wirbeltier ein Mensch ist, bevorzugt ein immungeschwächter Patient.

3. Oleylphosphocholin zur Verwendung nach Anspruch 1 oder 2, wobei besagtes Oleylphosphocholin zur parenteralen, oralen oder topischen Verabreichung formuliert ist.

4. Oleylphosphocholin zur Verwendung nach Anspruch 1 oder 2, wobei besagte Mykose eine systemische Mykose ist und besagte Formulierung zur intravenösen oder oralen Verabreichung ist.

5. Oleylphosphocholin zur Verwendung nach Anspruch 1 oder 2, wobei besagte Mykose eine subkutane Mykose ist und besagte Formulierung zur topischen oder oralen Verabreichung ist.

6. Oleylphosphocholin zur Verwendung nach einem der Ansprüche 1 bis 5, wobei besagte Mykose aus der Gruppe ausgewählt ist, bestehend aus Candidämie, Aspergillose und Cryptococcose.

## Revendications

1. Oléyl-phosphocholine pour utilisation dans le traitement d'une mycose invasive ou virulente chez un vertébré, l'agent pathogène responsable de cette mycose étant choisi dans le groupe constitué de *Candida albicans, Candida parapsilosis, Candida glabrata, Candida krusei, Aspergillus fumigatus, Aspergillus terreus, Fusarium solani, Scedosporium prolifacans, Cryptococcus neoformans, Microsporum canis, Sporothrix schenkii, Trichophyton rubrum, Trichophyton mentagrophytes* et *Fusarium oxysporum.*

2. Oléyl-phosphocholine pour utilisation selon la revendication 1, dans laquelle ledit vertébré est un être humain, de préférence un patient immunodéprimé.

3. Oléyl-phosphocholine pour utilisation selon la revendication 1 ou 2, dans laquelle ladite oléyl-phosphocholine est formulée pour une administration parentérale, orale ou topique.

4. Oléyl-phosphocholine pour utilisation selon la revendication 1 ou 2, dans laquelle ladite mycose est une mycose systémique et ladite formulation est destinée à une administration intraveineuse ou orale.

5. Oléyl-phosphocholine pour utilisation selon la revendication 1 ou 2, dans laquelle ladite mycose est une mycose sous-cutanée et ladite formulation est destinée à une administration topique ou orale.

6. Oléyl-phosphocholine pour utilisation selon l'une quelconque des revendications 1 à 5, dans laquelle ladite mycose est choisie dans le groupe constitué par la candidémie, l'aspergillose et la cryptococcose.
